# EUROPÄISCHE PATENTSCHRIFT

(11) **EP 1 296 965 B1**
(45) Veröffentlichungstag und Bekanntmachung des Hinweises auf die Patenterteilung: **05.10.2005**
(21) Anmeldenummer: 01947430.3
(22) Anmeldetag: 29.06.2001
(51) Int. Cl.: C07D 261/04, C07C 251/48, C07D 413/10, C07C 209/74, C07C 209/82, C07C 211/46

(54) **VERFAHREN ZUR HERSTELLUNG VON 4-BROM-ANILIN-DERIVATEN**
METHOD FOR PRODUCING 4-BROMINE-ANILINE DERIVATIVES
PROCEDE DE FABRICATION DE DERIVES DE LA 4-BROMO-ANILINE

(30) Priorität: 30.06.2000 DE 10030975
(43) Veröffentlichungstag der Anmeldung: 02.04.2003
(73) Patentinhaber: BASF Aktiengesellschaft, 67056 Ludwigshafen (DE)
(72) Erfinder: LOCHTMAN, Rene, 68161 Mannheim (DE); KEIL, Michael, 67251 Freinsheim (DE); GEBHARDT, Joachim, 67157 Wachenheim (DE); RACK, Michael, 69123 Heidelberg (DE); VON DEYN, Wolfgang, 67435 Neustadt (DE)
(86) Internationale Anmeldenummer: PCT/EP2001/007482
(87) Internationale Veröffentlichungsnummer: WO 2002/002537

(56) Entgegenhaltungen:
- WO-A-99/58509

## Beschreibung

Gegenstand der vorliegenden Erfindung ist ein Verfahren zur Herstellung von 4-Brom-Anilin-Derivaten.

4-Brom-Anilin-Derivate sind wertvolle Verbindungen, die als Zwischenprodukte in der chemischen Industrie verwendet werden. Sie eignen sich beispielsweise zur Herstellung von Wirkstoffen, die im Bereich des Pflanzenschutzes eingesetzt werden oder zur Herstellung von pharmazeutischen Wirkstoffen. Beispielsweise werden in WO 99/58509 Verfahren zur Herstellung von Isoxazolin-3-yl-acylbenzolen beschrieben, in denen 4-Brom-Anilin-Derivate als Zwischenprodukte zur Herstellung von herbiziden Wirkstoffen eingesetzt werden. In WO 98/31681 sind diese Wirkstoffe (2-Alkyl-3-(4,5-dihydroisoxazol-3-yl)-acylbenzole) als herbizide Wirkstoffe beschrieben.

Aus der Literatur ist bekannt, dass die selektive Bromierung von Anilinen in para-Stellung nicht oder nur mit Schwierigkeiten möglich ist (Houben-Weyl 5/4, 241, 274 ff). Die Bromierung mit elementarem Brom erfolgt in der Regel nicht selektiv, sondern ist häufig mit der Bildung von erheblichen Mengen an Dibromverbindungen verbunden. Die Selektivitäten zwischen Monobrom- zu Dibromverbindungen liegen erfahrungsgemäß in der Größenordnung von etwa 9:1, d.h. der Anteil an unerwünschten Dibromverbindungen liegt bei etwa 10 %. Lediglich mit sehr teuren Reagenzien, wie z.B. Tetrabutylammoniumtribromid, wurde so zum Beispiel bei -30°C die Verbindung 4-Brom-2-(4,5-dihydroisoxazol-3-yl)-3-methyl-anilin in einer Ausbeute von etwa 50 % erhalten (vgl. WO 99/58509).

Aufgabe der vorliegenden Erfindung war es, ein alternatives Verfahren zur Herstellung von 4-Brom-Anilin-Derivaten zur Verfügung zu stellen. Das in WO 99/58509 beschriebene Herstellungsverfahren des 4-Brom-2-(4,5-dihydroisoxazol-3-yl)-3-methyl-anilin-Derivates liefert nicht zufriedenstellende Ausbeuten und eine nicht zufriedenstellende Reinheit der Produkte. Insofern ist das in WO 99/58509 beschriebene Verfahren für die großtechnische Herstellung derartiger Verbindungen nur bedingt geeignet.

Gegenstand der vorliegenden Erfindung ist ein Verfahren zur Herstellung von 4-Brom-Anilin-Derivaten der Formel I 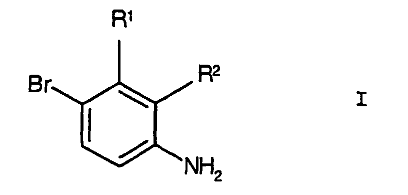 wobei die Substituenten folgende Bedeutung haben:
- R¹: C₁-C₆-Alkyl, C₁-C₆-Halogenalkyl, C₁-C₆-Alkoxy, C₁-C₆-Halogenalkoxy, C₃-C₈-Cycloalkyl, Halogen,
- R²: C₁-C₆-Alkyl, C₁-C₆-Alkoxy, C₃-C₈-Cycloalkyl, C₂-C₆-Alkenyl, Cyano oder ein heterocyclischer Rest,
dadurch gekennzeichnet, daß man eine Verbindung der Formel II 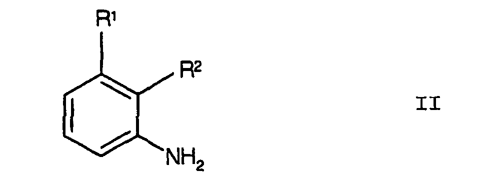 in der R¹ und R² die oben genannten Bedeutungen haben, mit einem Bromierungsmittel in Pyridin als Lösungsmittel oder in einem Lösungsmittelgemisch mit mindestens 55 Gew.-% an Pyridin umsetzt.

Mit Hilfe des erfindungsgemäßen Verfahrens können die Anilin-Derivate der Formel I in höheren Ausbeuten erhalten werden als dies bei den bisher bekannten Herstellungsverfahren der Fall ist. So kann beispielsweise nach dem in WO 99/58509 beschriebenen Verfahren (vgl. dort Beispiel 10) die Verbindung 4-Brom-2-(4,5-dihydroisoxazol-3-yl)-3-methyl-anilin in nur 47 % Ausbeute erhalten werden, während die Ausbeute nach dem erfindungsgemäßen Verfahren bei mindestens 60 %, vorzugsweise mindestens 70 % bzw. 80 %, und insbesondere mindestens 90 % liegt.

Außerdem werden die Verbindungen der Formel I in höherer Reinheit erhalten. Die Bromierung erfolgt hierbei mit einer hohen Selektivität in 4-Stellung des Phenylringes. Die Selektivität (Verhältnis Monobrom- zu Dibromverbindung) beträgt mindestens 92:8, insbesondere mindestens 95:5. Überraschenderweise liegt der Anteil an Verunreinigungen, wie z.B. von Dibromiden (bei diesen Dibromiden handelt es sich um Derivate der Formel I, die in 5- oder 6-Stellung durch ein weiteres Bromatom substituiert sind), die aus der entstehenden Reaktionsmischung nur schwer abtrennbar sind bzw. hierzu einen relativ hohen technischen Aufwand erfordern, bei unterhalb von 5 %. Die Anzahl weiterer zusätzlicher Reinigungsschritte zur Isolierung und Aufarbeitung der nach dem erfindungsgemäßen Verfahren hergestellten Verbindungen I kann daher reduziert werden. Dies ist insbesondere von Vorteil bei der großtechnischen Herstellung der Verbindungen I, da hierdurch ein effizientes und kostengünstiges Verfahren zur Verfügung gestellt werden kann.

Aufgrund der hohen Selektivität und des geringen Anteils an Dibromverbindungen kann das Reaktionsprodukt gegebenenfalls auch ohne zusätzliche Aufreinigung in die nächsten Verfahrensstufen für die weitere Umsetzung zu geeigneten Endprodukten übernommen werden.

C₁-C₆-Alkyl bedeutet eine geradkettige oder verzweigte Alkylgruppe mit 1 - 6 C-Atomen, wie z.B. Methyl, Ethyl, n-Propyl, iso-Propyl, n-Butyl, iso-Butyl, tert.-Butyl, n-Pentyl oder n-Hexyl; bevorzugt ist C₁-C₄-Alkyl, wie z.B. Methyl, Ethyl, n-Propyl, iso-Propyl, n-Butyl, iso-Butyl oder tert.-Butyl.

C₁-C₆-Halogenalkyl bedeutet eine geradkettige oder verzweigte C₁-C₆-Alkylgruppe wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl, Nonafluorbutyl, 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl; bevorzugt ist C₁-C₄-Halogenalkyl, wie Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
C₁-C₆-Alkoxy bedeutet eine geradkettige oder verzweigte Alkylgruppe mit 1 - 6 C-Atomen, wie z.B. Methoxy, Ethoxy, n-Propyloxy, iso-Propyloxy, n-Butyloxy, iso-Butyloxy, tert.-Butyloxy, n-Pentyloxy oder n-Hexyloxy; bevorzugt ist C₁-C₄-Alkoxy wie z.B. Methoxy, Ethoxy, n-Propyloxy, n-Butyloxy, iso-Butyloxy oder tert.-Butyloxy;
C₁-C₆-Halogenalkoxy bedeutet eine geradkettige oder verzweigte C₁-C₆-Alkoxygruppe wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2,difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy, Nonafluorbutoxy, 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy; bevorzugt ist C₁-C₄-Halogenalkoxy, wie Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2,difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
C₃-C₈-Cycloalkyl bedeutet einen unsubstituierten oder substituierten Cycloalkylring mit 3 - 8, wie z.B. Cyclopropyl, Cyclobutyl, Cyclopentyl, Cyclohexyl, Cycloheptyl oder Cyclooctyl. Als Substituenten kommen beispielsweise in Frage: C₁-C₆-Alkyl, C₁-C₆-Alkoxy oder Halogen;
bevorzugt ist C₃-C₆-Cycloalkyl, das umsubstituiert ist, wie z.B. Cyclopropyl, Cyclopentyl oder Cyclohexyl;
C₂-C₆-Alkenyl bedeutet eine geradkettige oder verzweigte Alkenylgruppe mit 2 - 6 C-Atomen, wobei die Doppelbindung an der Verknüpfungsposition sitzt, wie z.B. Ethenyl, Prop-1-en-1-yl, 1-Methylethenyl, Buten-1-yl, 1-Methyl-prop-1-en-1-yl, 2-Methyl-prop-1-en-1-yl, Penten-1-yl, 1-Methyl-but-1-en-1-yl, 2-Methyl-but-1-en-1-yl, 3-Methyl-but-1-en-1-yl, 1,2-Dimethyl-prop-1-en-1-yl, Hex-1-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1,2-Dimethyl-but-1-en-1-yl, 1,3-Dimethyl-but-1-en-1-yl, 2,3-Dimethyl-but-1-en-1-yl, 3,3-Dimethyl-but-1-en-1-yl, 1-Ethyl-but-1-en-1-yl, 2-Ethyl-but-1-en-1-yl oder 1-Ethyl-2-methyl-prop-1-en-yl;
Halogen bedeutet Fluor, Chlor, Brom, insbesondere Chlor oder Brom.

"Heterocyclischer Ring" bedeutet einen gesättigten, ungesättigten oder teilweise ungesättigten Heterocyclus mit 3 - 8 Ringatomen und einem, zwei oder drei Sauerstoff-, Schwefel- oder Stickstoffatomen. Bevorzugt sind Heterocyclen, die mindestens ein Sauerstoff- und/oder ein Stickstoffatom enthalten. Bevorzugt sind ferner Heterocylen mit 5 oder 6 Ringatomen. Die Verknüpfung des Heterocylus an den Phenylring kann an jeder beliebigen Stelle des Heterocyclus erfolgen, z.B. über ein heterocylisches N-Ringatom oder ein C-Ringatom. Die Heterocyclen sind unsubstituiert oder ein-, zwei- oder dreifach substituiert. Als Substituenten kommen solche Reste in Frage, die unter den gewählten Reaktionsbedingungen chemisch inert sind, wie z.B. C₁-C₆-Alkyl, C₁-C₆-Alkoxy oder Halogen. Im Sinne der vorliegenden Erfindung kommen als heterocyclische Ringe beispielsweise folgende Heterocyclen in Frage: Pyrrolyl, Pyrazolyl, Imidazolyl, Oxazolyl, Isoxazolyl, Thiazolyl, Thiadiazolyl, Piperidinyl, Morpholinyl, Oxazinyl, Isoxazolinyl, Isoxazolidinyl, etc.. Bevorzugt sind folgende Heterocyclen: Isoxazolyl, Isoxazolinyl oder Isoxazolidinyl, insbesondere 4,5-Dihydroisoxazol-3-yl oder 4,5-Dihydroisoxazol-5-yl.

Das erfindungsgemäße Verfahren eignet sich bevorzugt zur Herstellung von Verbindungen der Formel I, wobei die Substituenten folgende Bedeutung haben:
- R¹: C₁-C₆-Alkyl, C₁-C₆-Alkoxy, C₃-C₈-Cycloalkyl, Halogen
- R²: C₁-C₆-Alkyl, C₁-C₆-Alkoxy, C₃-C₈-Cycloalkyl, Cyano oder ein heterocyclischer Rest.

Das erfindungsgemäße Verfahren eignet sich weiterhin bevorzugt zur Herstellung der folgenden Verbindungen der Formel I:
4-Brom-2-(4,5-dihydroisoxazol-3-yl)-3-methylanilin,
4-Brom-2-(4,5-dihydroisoxazol-3-yl)-3-ethylanilin,
4-Brom-2-(4,5-dihydroisoxazol-3-yl-)3-methoxyanilin,
4-Brom-2-(4,5-dihydroisoxazol-3-yl)-3-ethoxyanilin,
4-Brom-2-(3-methyl-4,5-dihydroisoxazol-5-yl)-3-methylanilin,
4-Brom-2-(3-methyl-4,5-dihydroisoxazol-5-yl)-3-ethylanilin,
4-Brom-2-(3-methyl-4,5-dihydroisoxazol-5-yl-)3-methoxyanilin,
4-Brom-2-(3-methyl-4,5-dihydroisoxazol-5-yl)-3-ethoxyanilin,
4-Brom-2-(isoxazol-3-yl)-3-methylanilin,
4-Brom-2-(isoxazol-3-yl)-3-ethylanilin,
4-Brom-2-(isoxazol-3-yl-)3-methoxyanilin,
4-Brom-2-(isoxazol-3-yl)-3-ethoxyanilin,
4-Brom-2-(5-methyl-isoxazol-3-yl)-3-methylanilin,
4-Brom-2-(5-methyl-isoxazol-3-yl)-3-ethylanilin,
4-Brom-2-(5-methyl-isoxazol-3-yl-)3-methoxyanilin,
4-Brom-2-(5-methyl-isoxazol-3-yl)-3-ethoxyanilin,
4-Brom-2-cyano-3-methylanilin,
4-Brom-2-cyano-3-methoxyanilin.

Die Umsetzung der Verbindungen II mit einem Bromierungsmittel erfolgt vorzugsweise nach den folgenden Verfahrensschritten:

Die Umsetzung erfolgt erfindungsgemäß in Pyridin als Lösungsmittel oder in Lösungsmittelgemischen mit einem Anteil von mindestens 55 Gew.-, vorzugsweise 80 Gew.-%, an Pyridin. Bei Lösungsmittelgemischen eignen sich als weitere zusätzliche Lösungsmittel im Gemisch mit Pyridin beispielsweise Alkohole wie Methanol oder Ethanol, insbesondere Methanol; Ester wie Essigester oder Butylacetat, insbesondere Essigsäureethylester oder Butylacetat; Amide, wie beispielsweise N,N-Dimethylformamid oder N,N-Dimethylacetamid; oder Wasser.

Zunächst wird die Verbindung II in Pyridin oder einem Lösungsmittelgemisch enthaltend Pyridin als Lösung oder Suspension vorgelegt. Anschließend wird das Bromierungsmittel über einen Zeitraum von 5 Minuten - 5 Stunden zugegeben. Die Zugabe des Bromierungsmittels erfolgt entweder direkt, d.h. ohne Lösungsmittel, oder zusammen mit einem geeigneten Lösungsmittel.

Als Bromierungsmittel eignen sich vorzugsweise elementares Brom oder eine Mischung von HBr und Wasserstoffperoxid. Im Falle von Brom erfolgt die Zugabe vorzugsweise zusammen mit einem geeigneten Lösungsmittel, wie z.B. Pyridin unter Bildung von Pyridiniumperbromid. In diesem Falle wird eine besonders hohe Selektivität im Verhaltnis Monobrom- zu Dibromverbindung erzielt.

In einer bevorzugten Ausführungsform des Verfahrens werden das Bromierungsmittel und die Verbindung II in einem Molverhältnis von 1:1 bis 2:1 eingesetzt. Vorzugsweise wird das Bromierungsmittel äquimolar oder im geringen Überschuß eingesetzt.

Die Umsetzung erfolgt bei Temperaturen von 20 °C bis zum Siedepunkt des Lösungsmittels, vorzugsweise im Bereich zwischen 60 °C und 85 °C. In einer weiteren bevorzugten Ausführungsform arbeitet man bei Temperaturen von 50°C bis 100°C, vorzugsweise im Bereich zwischen 80°C und 100°C. Insbesondere bevorzugt bei etwa 100°C.

Die Reaktionszeit beträgt 1 - 24 Stunden, vorzugsweise 2 - 12 Stunden, insbesondere 5 - 8 Stunden. In einer weiteren bevorzugten Ausführungsform beträgt die Reaktionszeit 30 min bis 10 h, vorzugsweise 30 min bis 5 Stunden.

Für den Fall, daß eine Mischung von HBr und Wasserstoffperoxid als Bromierungsmittel verwendet wird, erfolgt die Zugabe des Bromierungsmittels zur Lösung von II über einen Zeitraum von vorzugsweise 10 Minuten bis 3 Stunden. Das Molverhältnis von HBr zur Verbindung II liegt vorzugsweise im Bereich von 1:1 bis 1,5:1. Die Zugabe erfolgt bei Temperaturen von 0 - 50 °C, vorzugsweise zwischen 20 - 40 °C . Anschließend erfolgt die Zugabe von Wasserstoffperoxid. Das Molverhältnis von H₂O₂ zu HBr beträgt 1:1 bis 1,5:1. Die Zugabe erfolgt bei Temperaturen von 10 °C bis zum Siedepunkt des Lösungsmittels, vorzugsweise zwischen 50°C und 120°C, bevorzugt zwischen 80°C und 100°C, insbesondere bevorzugt bei etwa 100°C; in einer weiteren bevorzugten Ausführungsform erfolgt die Zugabe vorzugsweise zwischen 60 °C und 85 °C. Anschließend wird die Lösung über einen Zeitraum von 10 min - 36 Stunden, vorzugsweise 10 min - 8 Stunden, nachgerührt. In einer weiteren Ausführungsform wird die Lösung über einen Zeitraum von 1 - 36 Stunden, vorzugsweise von 2 - 8 Stunden nachgerührt. In einer weiteren Ausführungsform wird die Lösung über einen Zeitraum von 10 min bis 3 Stunden, vorzugsweise 10 min bis 2 Stunden nachgerührt. Anschließend erfolgt die Aufarbeitung und Reinigung des Produktes. Hierzu wird die Lösung eingeengt. Das Rohprodukt wird in einem geeigneten Lösungsmittel, vorzugsweise Pyridin oder ein Lösungsmittelgemisch enthaltend mindestens 50 % Pyridin, gelöst und mit Wasser versetzt. Nach Filtration und Waschen des Rückstandes bzw. Kristallisation unter Verwendung eines geeigneten Lösungsmittel (z.B. Wasser) wird das Produkt in guter Ausbeute und hoher Reinheit erhalten.

Es ist aber auch möglich das Rohprodukt in Dimethyldisulfid aufzunehmen und mit Wasser und/oder Natronlauge zu waschen. Die organische Lösung kann dann in Folgereaktionen eingesetzt werden.

In einer bevorzugten Ausführungsform wird die Verbindung der Formel II in Pyridin oder einem Gemisch von Pyridin und Wasser vorgelegt. In letzterem Fall liegt das Verhältnis von Pyridin zu Wasser im Bereich von 80 bis 98 Gew.-% zu 20 bis 5 Gew.-%; vorzugsweise in einem Bereich von 90 bis 95 Gew.-% zu 10 bis 5 Gew.-%.

Das Verhältnis von der Verbindung der Formel II zu Pyridin bzw. Pyridin/Wasser wird so gewählt, daß eine 5 bis 25 %ige Lösung entsteht, vorzugsweise eine 10 bis 15 %ige Lösung. Zu der resultierenden Lösung werden nun etwa 0,8 bis 1,1 mol-Äquivalente, vorzugsweise 0,9 bis 1,0 mol Äquivalente HBr zugegeben. Nach Entfernen des Wassers durch azeotrope Destillation wird zu der verbleibenden Lösung innerhalb eines Zeitraums von 1 bis 3 Stunden, vorzugsweise 1,5 bis 2,5 Stunden bei 50 bis 120°C, vorzugsweise bei 80 bis 110°C, insbesondere bei etwa 100°C Wasserstoffperoxid zugegeben. Das Wasserstoffperoxid wird üblicherweise in einer 20 %iger bis 50 %iger, vorzugsweise 30 bis 50 %iger wäßriger Lösung eingesetzt.

Anschließend wird etwa 10 min bis 2 h, vorzugsweise 30 min bis 1 h nachgerührt.

Nun schließt sich die Aufarbeitung des Produktes an. Hierzu wird die Reaktionslösung auf etwa Raumtemperatur abgekühlt, ggf. mit wäßriger Natriumsulfit-Lösung gewaschen und die organische Phase eingeengt. Das so erhaltene Produkt kann ohne weitere Reinigung in Folgereaktionen eingesetzt werden. Es ist aber auch möglich den Rückstand in Dimethyldisulfid aufzunehmen, die resultierende Lösung mit Wasser oder Natronlauge zu waschen und die resultierende organische Phase in Folgereaktionen einzusetzen.

In einer weiteren Ausführungsform ist es möglich das Pyridin, das ggf. bis zu 10 % Wasser enthält, mit der HBr zu versetzen und anschließend das Wasser azeotrop zu entfernen. In dem Reaktionsgemisch wird dann die Verbindung der Formel II gelöst und das Wasserstoffperoxid zugetropft. Sowohl die Mengenverhältnisse der eingesetzten Stoffe als auch die Zeit- und Temperaturführung entsprechen den obengenannten Bedingungen.

In einer weiteren Ausführungsform ist es auch möglich anstelle von Pyridin und HBr Pyridiniumhydrobromid zu verwenden.

Für den Fall, daß elementares Brom als Bromierungsmittel verwendet wird, erfolgt die Zugabe des Bromierungsmittels zur Lösung von II vorzugsweise portionsweise oder kontinuierlich über einen Zeitraum von etwa 30 Minuten bis 6 Stunden. Das Molverhältnis von Brom zur Verbindung II liegt vorzugsweise im Bereich von 1:1 bis 1,5:1. Die Zugabe erfolgt bei Temperaturen von 0 - 50 °C, vorzugsweise bei Raumtemperatur. Anschließend wird die Lösung über einen Zeitraum von 1 - 24 Stunden, vorzugsweise 2 - 8 Stunden, nachgerührt. Anschließend erfolgt die Aufarbeitung und Reinigung des Produktes. Hierzu wird die Lösung eingeengt. Das Rohprodukt wird in einem geeigneten Lösungsmittel, vorzugsweise Pyridin oder ein Lösungsmittelgemisch enthaltend mindestens 50 % Pyridin, gelöst und mit Wasser versetzt. Nach Filtration und Waschen des Rückstandes bzw. Kristallisation unter Verwendung eines geeigneten Lösungsmittel (z.B. Wasser) wird das Produkt in guter Ausbeute und hoher Reinheit erhalten.

Das Produkt kann ferner auch durch Extraktion aus der Reaktionslösung erhalten werden. Hierzu wird die Reaktionslösung zunächst eingeengt und der Rückstand mit einem geeigneten Lösungsmittel oder Lösungsmittelgemisch, wobei die Komponenten beispielsweise ausgewählt sind aus Wasser, Essigester und Dimethyldisulfid (DMDS), insbesondere Wasser, Essigester, Wasser/Essigester oder Wasser/DMDS, aufgenommen. Für die Extraktion eignen sich mit Wasser nicht mischbare Lösungsmittel oder entsprechende Lösungsmittelgemische, wie zum Beispiel Essigester, Butylacetat, Toluol oder Methyl-tert.-butylether (MTBE). Nach Einengen der Lösung wird das Produkt in guter Ausbeute und hoher Reinheit erhalten.

Die Reinigung des Rohproduktes erfolgt entweder durch Waschen des erhaltenen Rückstandes oder durch Kristallisation. Zum Waschen eignen sich beispielsweise Wasser oder wässrige Lösungsmittel. Zur Umkristallisation eignen sich beispielsweise Toluol oder Benzol.

Grundsätzlich kann das erhaltene Rohprodukt auch ohne weitere Aufreinigung der Reaktionslösung für die nächste Reaktionsstufe im Rahmen der weiteren Umsetzung zur Herstellung von Wirkstoffen eingesetzt werden. Die Reaktionslösung enthaltend Verbindungen der Formel I kann hierzu mit weiteren Lösungsmitteln verdünnt werden und so als Rohlösung für die nächste Verfahrensstufe eingesetzt werden. Alternativ kann die Reaktionslösung auch eingeengt werden und der erhaltene Rückstand direkt oder als Schmelze in die nächste Verfahrensstufe überführt werden.

Die als Ausgangsmaterialien einzusetzenden Verbindungen der Formel II sind literaturbekannt bzw. käuflich erhältlich. Sie können nach an sich bekannten Verfahren hergestellt werden, wie z.B. in WO 98/31681 oder WO 99/58509 näher beschrieben.

In den folgenden Ausführungsbeispielen wird die Erfindung näher erläutert.

### Beispiel 1

### 4-Brom-2-(4,5-dihydroisoxazol-3-yl)-3-methylanilin

### Bromierungsmittel: HBr/H₂O₂

100,5 g 2-(4,5-dihydroisoxazol-3-yl)-3-methylanilin in 2000 g Pyridin vorlegen und 98,2 g HBr bei 20-35°C zutropfen. Bei einer Temperatur von 78-84°C wird anschließend 64,6 g Wasserstoffperoxid innerhalb von 0,5 h zugetropft. Es werden weitere 12 Stunden bei 25°C nachgerührt. Danach wird eingeengt bis ein öliger Rückstand übrig bleibt. Das Rohprodukt wird in 100 ml Pyridin bei 50°C gelöst und mit 1000 ml Wasser versetzt. Nach 1 h rühren bei 0°C wird abfiltriert, zweimal mit 200 ml Wasser gewaschen und getrocknet.

Man erhält 141 g (Ausbeute: 92%) eines gelben Feststoffes (HPLC: 94.6% 4-Brom-2-(4,5-dihydroisoxazol-3-yl)-3-methylanilin, 1.8% 6-Brom-2-(4,5-dihydroisoxazol-3-yl)-3-methylanilin, 3.4% 4,6-Dibrom-2-(4,5-dihydroisoxazol-3-yl)-3-methylanilin).

### Beispiel 2

### 4-Brom-2-(4,5-dihydroisoxazol-3-yl)-3-methylanilin

### Bromierungsmittel: Brom

100 g 2-(4,5-dihydroisoxazol-3-yl)-3-methylanilin in 1000 g Pyridin vorlegen und innerhalb 3 Stunden eine Lösung von insgesamt 96,19 g Brom in 1000 g Pyridin bei 20°C in fünf frisch hergestellten Portionen zutropfen. Es werden.weitere 12 Stunden nachgerührt. Pyridin bei 150 mbar und einer Badtemperatur von 75°C abdestillieren. Der Rückstand wird in 2 l Wasser gelöst und mehrmals mit je 250 ml Essigester extrahiert. Nach Einengen erhält man 122,1 g Produkt (Ausbeute 81,6%; GC: 93.2% 4-Brom-2-(4,5-dihydroisoxazol-3-yl)-3-methylanilin, 2.7% 6-Brom-2-(4,5-dihydroisoxazol-3-yl)-3-methylanilin, 4.1% 4,6-Dibrom-2-(4,5-dihydroisoxazol-3-yl)-3-methylanilin).

### Beispiel 3

### 4-Brom-2-(4,5-dihydroisoxazol-3-yl)-3-methylanilin

### Bromierungsmittel: Brom

5 g der Verbindung 2-(4,5-Dihydroisoxazol-3-yl)-3-methylanilin wird in 50 g Pyridin vorgelegt und bei 20°C innerhalb 5 h eine Lösung von insgesamt 4,89 g Brom in 50 g Pyridin (Mischung bei 0°C herstellen) zugetropft. Es werden weitere 12 Stunden bei 25°C nachgerührt. Der Ansatz wird in 250 ml Wasser gegossen und dreimal mit je 100 ml MTBE extrahiert. Die vereinigten organischen Phasen werden zweimal mit je 100 ml Wasser gewaschen, über Natriumsulfat getrocknet und eingeengt.

Man erhält 6.0 g Produkt (Ausbeute 79.8%; 94.3% 4-Brom-2-(4,5-dihydroisoxazol-3-yl)-3-methylanilin, 1.8% 6-Brom-2-(4,5-dihydroisoxazol-3-yl)-3-methylanilin, 3.5% 4,6-Dibrom-2-(4,5-dihydroisoxazol-3-yl)-3-methylanilin).

### Beispiel 4

### 4-Brom-2-(4,5-dihydroisoxazol-3-yl)-3-methylanilin

### Bromierungsmittel: HBr, H₂O₂

500,0 g 2-(4,5-Dihydroisoxazol-3-yl)-3-methylanilin werden in 4500 g Pyridin vorgelegt und 467,4 g 47%ige HBr bei 25 35 °C zugetropft. Unter Rückfluß wird das Wasser bei Normaldruck azeotrop abdestilliert. Anschließend wird bei 100°C 199,2 g 50 %iges H₂O₂ innerhalb von 2 Stunden zugetropft. Nach 1 Stunde nachrühren kühlt man auf Raumtemperatur ab, wäscht das Reaktionsgemisch mit Natriumsulfit-Lösung und destilliert dann das Lösungsmittel ab (T<100°C). Der Rückstand wird in 3220 g Dimethyldisulfid aufgenommen und bei 60°C mit 1500 g Wasser gewaschen. Die resultierende Lösung wird in einer Folgereaktion eingesetzt.

Man erhält etwa 83 % an dem gewünschten Produkt.

## Patentansprüche

1. Verfahren zur Herstellung von 4-Brom-Anilin-Derivaten der Formel I 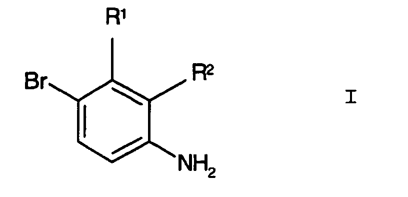 wobei die Substituenten folgende Bedeutung haben:
R¹ C₁-C₆-Alkyl, C₁-C₆-Halogenalkyl, C₁-C₆-Alkoxy, C₁-C₆-Halogenalkoxy, C₃-C₈-Cycloalkyl, Halogen
R² C₁-C₆-Alkyl, C₁-C₆-Alkoxy, C₃-C₈-Cycloalkyl, C₂-C₆-Alkenyl, Cyano oder ein heterocyclischer Rest,
**dadurch gekennzeichnet, daß** man eine Verbindung der Formel II 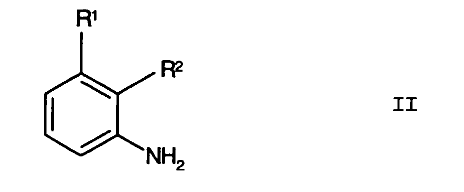 in der R¹ und R² die oben genannten Bedeutungen haben, mit einem Bromierungsmittel in einem Lösungsmittelgemisch mit einem Anteil von mindestens 55 Gew.-% an Pyridin umsetzt.

2. Verfahren nach Anspruch 1, **dadurch gekennzeichnet, daß** als Bromierungsmittel Brom verwendet wird.

3. Verfahren nach Anspruch 1, **dadurch gekennzeichnet, daß** als Bromierungsmittel Bromwasserstoff und Wasserstoffperoxid verwendet wird.

4. Verfahren nach den Ansprüchen 1 - 3, **dadurch gekennzeichnet, daß** als Lösungsmittel Pyridin eingesetzt wird.

5. Verfahren nach einem der Ansprüche 1 - 4, wobei R¹ C₁-C₆-Alkyl ist.

6. Verfahren nach Anspruch 5, wobei R¹ Methyl oder Ethyl ist.

7. Verfahren nach einem der Ansprüche 1 - 6, wobei R² ein heterocyclischer Ring ist.

8. Verfahren nach Anspruch 7, wobei R² ein Isoxazol-, Isoxazolin- oder Isoxazolidin-Ring ist.

9. Verfahren nach Anspruch 8, wobei R² 4,5-Dihydroisoxazol-3-yl oder 4,5-Dihydroisoxazol-5-yl bedeutet.

10. Verfahren nach einem der Ansprüche 1 - 8 zur Herstellung von 4-Brom-2-(4,5-dihydroisoxazol-3-yl)-3-methyl-anilin.

## Claims

1. A process for preparing 4-bromoaniline derivatives of the formula I 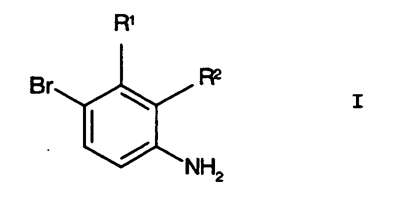 where:
R¹ is C₁-C₆-alkyl, C₁-C₆-haloalkyl, C₁-C₆-alkoxy, C₁-C₆-haloalkoxy, C₃-C₈-cycloalkyl, halogen
R² is C₁-C₆-alkyl, C₁-C₆-alkoxy, C₃-C₈-cycloalkyl, C₂-C₆-alkenyl, cyano or a heterocyclic radical,
which comprises reacting a compound of the formula II 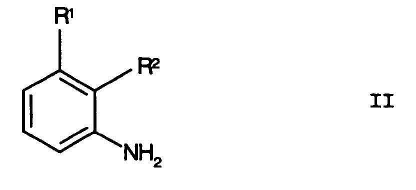 in which R¹ and R² are as defined above with a brominating agent in a solvent mixture comprising at least 55% by weight of pyridine.

2. A process as claimed in claim 1, wherein the brominating agent used is bromine.

3. A process as claimed in claim 1, wherein the brominating agent used is hydrogen bromide and hydrogen peroxide.

4. A process as claimed in any of claims 1 - 3, wherein the solvent used is pyridine.

5. A process as claimed in any of claims 1 - 4, where R¹ is C₁-C₆-alkyl.

6. A process as claimed in claim 5, where R¹ is methyl or ethyl.

7. A process as claimed in any of claims 1 - 6, where R² is a heterocyclic ring.

8. A process as claimed in claim 7, where R² is an isoxazole, isoxazoline or isoxazolidine ring.

9. A process as claimed in claim 8, where R² is 4,5-dihydroisoxazol-3-yl or 4,5-dihydroisoxazol-5-yl.

10. A process as claimed in any of claims 1 - 8 for preparing 4-bromo-2-(4,5-dihydroisoxazol-3-yl)-3-methylaniline.

## Revendications

1. Procédé de préparation de dérivés de 4-bromoaniline de la formule I 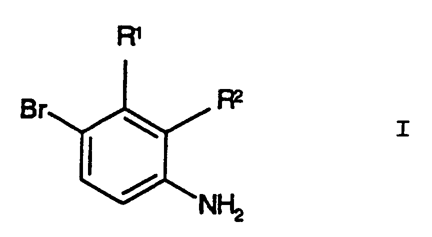 dans laquelle les substituants ont la signification suivante :
R¹ = C₁-C₆-alkyle, C₁-C₆-halogénoalkyle, C₁-C₆-alcoxy, C₁-C₆-halogénoalcoxy, C₃-C₈-cycloalkyle, halogène,
R² = C₁-C₆-alkyle, C₁-C₆-alcoxy, C₃-C₈-cycloalkyle, C₂-C₆-alcényle, cyano ou un radical hétérocyclique,
**caractérisé en ce qu'**on fait réagir un composé de la formule II 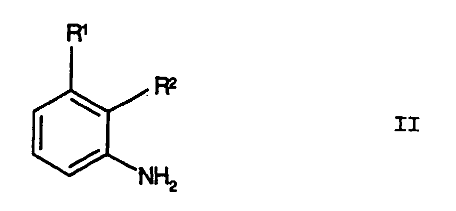 dans laquelle R¹ et R² ont les significations indiquées ci-dessus, avec un agent de bromation dans un mélange de solvants comportant une fraction d'au moins 55% en poids de pyridine.

2. Procédé suivant la revendication 1, **caractérisé en ce que**, comme agent de bromation, on utilise du brome.

3. Procédé suivant la revendication 1, **caractérisé en ce que**, comme agent de bromation, on utilise du bromure d'hydrogène et du peroxyde d'hydrogène.

4. Procédé suivant les revendications 1 à 3, **caractérisé en ce que**, comme solvant, on met en oeuvre de la pyridine.

5. Procédé suivant l'une des revendications 1 à 4, dans lequel R¹ est un alkyle en C₁-C₆.

6. Procédé suivant la revendication 5, dans lequel R¹ est du méthyle ou de l'éthyle.

7. Procédé suivant l'une des revendications 1 à 6, dans lequel R² est un noyau hétérocyclique.

8. Procédé suivant la revendication 7, dans lequel R² est un noyau d'isoxazole, d'isoxazoline ou d'isoxazolidine.

9. Procédé suivant la revendication 8, dans lequel R² représente du 4,5-dihydroisoxazol-3-yle ou du 4,5-dihydroisoxasol-5-yle.

10. Procédé suivant l'une des revendications 1 à 8, pour la préparation de 4-bromo-2-(4,5-dihydroisoxazol-3-yl)-3-méthyl-aniline.
